# FASCICULE DE BREVET EUROPEEN

(11) **EP 2 371 413 B1**
(45) Date de publication et mention de la délivrance du brevet: **15.08.2018**
(21) Numéro de dépôt: 11159527.8
(22) Date de dépôt: 24.03.2011
(51) Int. Cl.: A61M 16/06, A61M 16/08

(54) **Sonde nasale**
Nasale Sonde
Nasal probe

(30) Priorité: 31.03.2010 FR 1001323; 16.12.2010 FR 1004911
(43) Date de publication de la demande: 05.10.2011
(73) Titulaire: Boussignac, Georges, 92160 Antony (FR)
(72) Inventeur: Boussignac, Georges, 92160 Antony (FR)
(74) Mandataire: Jacobacci Coralis Harle

(56) Documents cités:
- WO-A1-92/20392
- WO-A1-98/48876
- WO-A1-2008/014543
- WO-A1-2009/151344
- WO-A2-03/041780
- DE-A1-102005 018 696
- FR-A1- 2 896 697
- US-A1- 2005 103 346
- US-A1- 2008 047 559
- US-A1- 2009 173 350

## Description

La présente invention concerne une sonde nasale, notamment destinée à acheminer du gaz respiratoire dans l'appareil respiratoire d'un patient.

Quoique non exclusivement, la sonde nasale conforme à la présente invention est tout particulièrement appropriée pour être utilisée en coopération avec des dispositifs d'oxygénothérapie administrant de façon continue ou discontinue de l'oxygène sous pression dans le but de maintenir ou de rétablir un taux constant d'oxygène dans le sang, ou bien encore avec des dispositifs d'assistance respiratoire utilisables sur des patients dont la respiration spontanée est absente ou insuffisante, qu'ils soient placés ou non sous respiration artificielle.

Pour cela, les sondes nasales utilisées comportent, de façon connue, deux conduits destinés à être introduits dans les narines respectives du nez d'un patient, de manière à pouvoir acheminer du gaz respiratoire dans l'appareil respiratoire de ce dernier. Ces sondes nasales sont ensuite raccordées aux dispositifs d'oxygénothérapie ou d'assistance respiratoire précités, par exemple par l'intermédiaire d'un raccord tubulaire.

Une telle sonde nasale, positionnée dans les narines d'un patient par un opérateur, est généralement maintenue en position par l'intermédiaire d'un lacet de maintien, souvent élastique, serré autour de la tête du patient.

Le maintien par serrage provoque fréquemment, en plus d'une gêne et d'un inconfort chez le patient, des traumatismes de la muqueuse des narines lorsque le serrage est excessif ou le positionnement de la sonde inadéquat.

Par ailleurs, les mouvements du patient provoquent des déplacements intempestifs de la sonde en position dans les narines, de sorte que les conduits heurtent ou frottent fortement la muqueuse de ces dernières. Cela conduit généralement à l'apparition de lésions (notamment des escarres) sur la muqueuse des narines, du fait de la rigidité desdits conduits. Les documents WO 2008/014543 A1, WO 2009/151344 A1, WO 92/20392 A1, US 2005/103346 A1, US 2008/047559 A1, DE 10 2005 018 696 A1 et WO 03/041780 A2 décrivent des sondes nasales connues de l'art antérieur. Le document WO 03/041780 A2 décrit une sonde nasale selon le préambule de la revendication 1.

La présente invention a pour objet de remédier aux inconvénients mentionnés ci-dessus.

A cette fin, selon l'invention, la sonde nasale pour acheminer du gaz respiratoire dans l'appareil respiratoire d'un patient comportant :
- deux conduits destinés à être introduits, au moins partiellement, dans les narines respectives du nez du patient et aptes à être alimentés par du gaz respiratoire ; et
- deux jupes latérales souples dont chacune d'elles entoure un desdits conduits, sur au moins une partie de sa longueur, en ménageant un espace annulaire entre elle et ledit conduit correspondant, lesdites jupes latérales étant destinées à être introduites, au moins en partie, dans les narines correspondantes du nez du patient,
est remarquable :
- en ce que chacun desdits conduits comporte au moins un orifice latéral qui est ménagé dans sa paroi latérale et qui est en regard de ladite jupe latérale associée ; et
- en ce que lesdites jupes latérales sont déformables sous l'effet d'un jet de gaz s'échappant dudit au moins un orifice latéral, de manière à être appliquées contre la paroi interne des narines correspondantes pour participer au maintien de ladite sonde nasale dans ces dernières.

Ainsi, grâce à l'invention, les jupes latérales souples participent au maintien de la sonde nasale dans les narines d'un patient. Le lacet de maintien habituellement associé à une sonde nasale pour la maintenir en position, peut, à défaut d'être complètement supprimé, au moins être desserré pour diminuer la gêne et l'inconfort occasionnés par le serrage dudit lacet autour de la tête du patient.

En outre, la jupe latérale est maintenue plaquée, une fois en position, contre la paroi interne de la narine correspondante par le jet de gaz respiratoire s'échappant du ou des orifices latéraux et écartant radialement la jupe en direction de la paroi interne. Autrement dit, le gaz traversant le ou les orifices latéraux étire la jupe qui se déforme légèrement et vient s'appliquer contre la paroi interne de la narine correspondante. Ainsi, l'étanchéité entre la paroi interne et la jupe latérale est garantie, ce qui permet le maintien d'une légère pression positive à l'intérieur des narines.

De plus, les jupes latérales souples permettent également la protection de la muqueuse des narines contre des lésions susceptibles d'être provoquées par les conduits de la sonde nasale, au cours de son utilisation, en formant des interfaces souples de protection.

Pour améliorer davantage la protection de la muqueuse des narines d'un patient, chacune desdites jupes latérales entoure, de préférence, l'extrémité distale dudit conduit associé, extrémité qui est la plus à même de provoquer des lésions de la muqueuse.

De préférence, chacune desdites jupes latérales souples est fabriquée en silicone.

De préférence encore, chacun desdits conduits comporte plusieurs orifices latéraux, ces derniers pouvant être répartis équi-angulairement autour d'une ou plusieurs sections dudit conduit, de manière à assurer une étanchéité maximale et uniforme entre la jupe latérale associée et la paroi interne de la narine correspondante. Pour améliorer le maintien de la sonde dans les narines d'un patient ainsi que l'étanchéité entre les jupes latérales et la paroi interne des narines, chacune desdites jupes latérales peut avantageusement présenter une section transversale, par rapport à l'axe longitudinal dudit conduit associé, au moins égale à la section transversale de la narine correspondante. Ainsi, la jupe latérale souple, une fois insérée dans la narine correspondante du patient, est en contact avec la paroi interne de celle-ci.

De préférence, pour chacun desdits conduits, ledit espace annulaire associé est ménagé au moyen d'une collerette annulaire, appartenant à la jupe latérale correspondante, qui est rapportée sur la surface latérale extérieure dudit conduit associé. Ainsi, on évite le collage de la jupe sur la surface latérale extérieure du conduit. En outre, lorsque les conduits comportent un ou plusieurs orifices latéraux, un tel espace annulaire facilite le passage du gaz (vicié et/ou respiratoire) au travers desdits orifices et contribue à la protection de la muqueuse des narines en maintenant les extrémités distales desdits conduits éloignées de la muqueuse.

Dans une forme de réalisation conforme à l'invention, chaque collerette est liée à l'extrémité proximale de la jupe latérale correspondante, fermant ainsi cette extrémité. Dans ce cas, le ou les orifices latéraux favorisent la création de turbulences au sein des narines, de sorte que les jets gazeux sortant des conduits atteignent la muqueuse des parois internes des narines, non pas directement, mais d'une façon diffuse moins agressive pour la muqueuse. Cela a pour effet de réduire sensiblement l'apparition de lésions sur la muqueuse.

Dans une autre forme de réalisation conforme à l'invention, chaque collerette est liée à l'extrémité distale de la jupe latérale correspondante, fermant ainsi cette extrémité. En cas de surpression gazeuse à l'intérieur d'une narine, du gaz en surpression peut venir décoller la jupe latérale correspondante appliquée contre la paroi interne en regard, de manière à former un passage lui permettant de s'échapper vers l'extérieur, abaissant ainsi la pression à l'intérieur de la narine. On prévient ainsi toute surpression à même de provoquer une gêne chez le patient, ou bien encore des lésions de la muqueuse.

Quelle que soit la forme de réalisation réalisée, la collerette et la jupe latérale ne forment, de préférence, qu'une seule et même pièce.

Avantageusement, les deux conduits de ladite sonde nasale sont reliés l'un à l'autre par l'intermédiaire d'un conduit de liaison, lui-même relié à un embout de raccordement.

Par ailleurs, la sonde nasale peut comporter des moyens de prélèvement de gaz vicié expiré par le patient qui sont destinés à être reliés à des dispositifs de mesure (analyseur de CO2, manomètre, ...).

A titre d'exemple, ces moyens de prélèvement peuvent se présenter sous la forme d'une ou plusieurs saillies tubulaires qui peuvent communiquant avec au moins un desdits conduits de la sonde nasale.

Selon une forme de réalisation de l'invention, l'extrémité distale des deux conduits de la sonde se présente sous la forme d'un tube comportant un canal d'acheminement des gaz. En variante, l'extrémité distale desdits conduits peut être en forme de tétine, dont la paroi arrondie convexe est percée d'une pluralité d'orifices permettant le passage des gaz.

Les figures du dessin annexé feront bien comprendre comment l'invention peut être réalisée. Sur ces figures, des références identiques désignent des éléments semblables.
La figure 1 est une vue schématique en coupe d'un premier exemple de sonde nasale conforme à la présente invention.
La figure 2 est une vue schématique du dessus de la sonde nasale de la figure 1.
La figure 3 représente, de façon schématique, la figure d'un patient équipé de la sonde nasale de la figure 1.
La figure 4, semblable à la figure 1, montre la sonde nasale de l'invention une fois introduite dans les narines d'un patient, conformément à la figure 3.
Les figures 5, 6 et 7, respectivement semblables aux figures 1, 2 et 3, représentent un second exemple de sonde nasale conforme à la présente invention.
La figure 8 est un agrandissement schématique de la zone G de la figure 6.

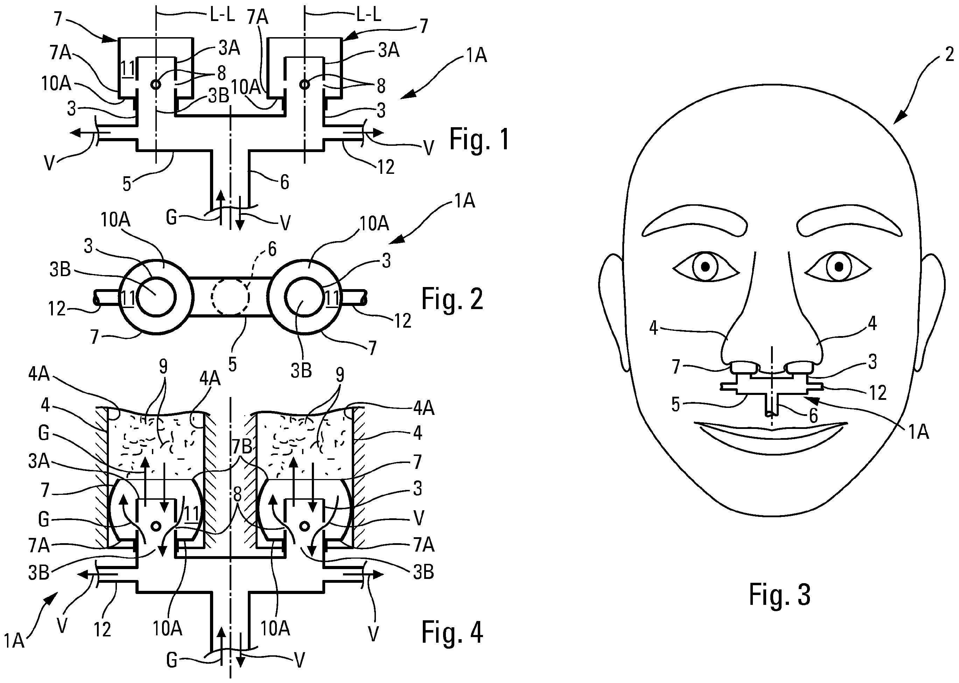

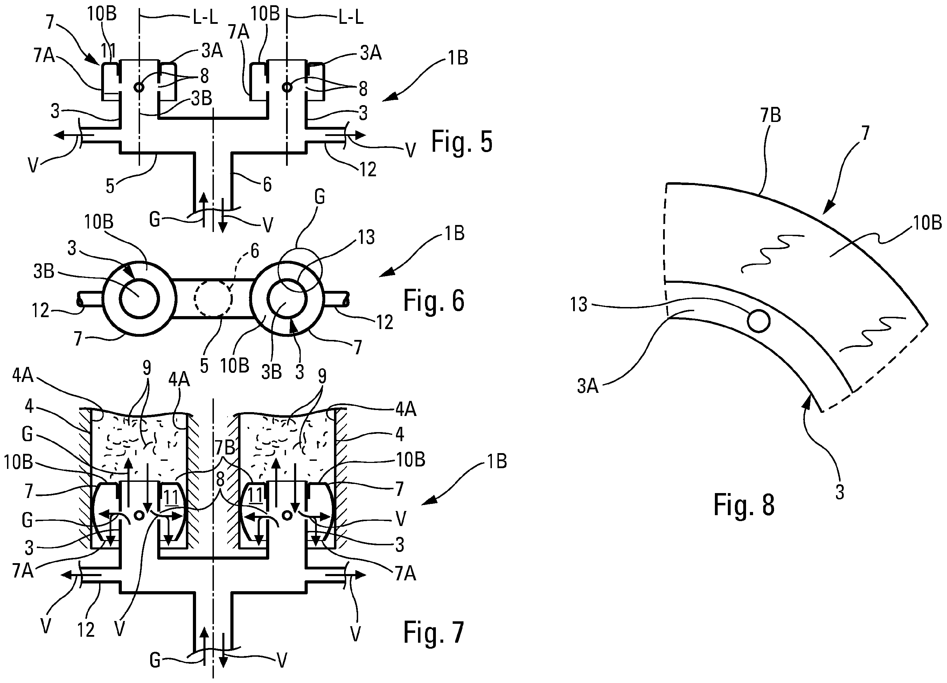

Sur les figures 1 et 5, on a représenté un premier et un second exemples 1A et 1B de sonde nasale, conforme à l'invention, destinée à être utilisée, quoique non exclusivement, en coopération avec des dispositifs d'oxygénothérapie (non représentés sur les figures) ou bien encore avec des dispositifs d'assistance respiratoire (non représentés) utilisables sur des patients dont la respiration spontanée est absente ou insuffisante, qu'ils soient placés ou non sous respiration artificielle.

Comme le montrent les figures 1 à 7, la sonde nasale 1A, 1B, destinée à acheminer du gaz respiratoire (par exemple de l'oxygène pur) dans l'appareil respiratoire d'un patient 2, comporte, de façon connue, deux conduits 3 tubulaires, dont au moins les extrémités distales 3A sont destinées à être introduites dans les narines 4 respectives du nez du patient 2.

Les conduits 3 comportent chacun un canal interne 3B permettant, d'une part, l'acheminement de gaz respiratoire (symbolisé par la flèche G) provenant d'une source de gaz respiratoire externe (non représentée sur les figures) et, d'autre part, l'évacuation du gaz vicié (représenté par la flèche V) expiré par le patient 2.

Dans les exemples des figures 1 à 7, les conduits 3 sont reliés l'un à l'autre par l'intermédiaire d'un conduit de liaison 5, lui-même relié à un embout de raccordement 6 raccordé à la source de gaz respiratoire.

Selon l'invention, la sonde 1A, 1B comporte deux jupes latérales 7 souples qui entourent chacune un des conduits 3, sur au moins une partie de sa longueur, en ménageant un espace annulaire 11 entre elles et ledit conduit 3 correspondant.

Par jupe latérale, on entend une surface de révolution qui est définie autour de l'axe longitudinal L-L d'un conduit 3 et qui présente, par exemple, la forme d'un cylindre, ou bien encore d'un tonneau, ouvert à ses extrémités longitudinales.

Chaque jupe latérale 7 est destinée à être introduite, au moins partiellement, dans la narine 4 correspondante du nez du patient 2. De cette façon, la jupe 7 participe au maintien de la sonde nasale 1A, 1B dans les narines 4 du patient 2.

En outre, des orifices latéraux 8 sont répartis équi-angulairement autour d'une même section du conduit 3, au niveau de son extrémité distale 3A. Ces orifices 8 sont ménagés dans la paroi du conduit 3. Bien entendu, la présente invention n'est nullement limitée à une telle répartition des orifices, toute autre disposition appropriée étant également envisageable (répartition longitudinale, aléatoire, etc.).

De plus, les orifices 8 de chacun des conduits 3 sont en regard de la jupe latérale 7 associée, ce qui permet, une fois la sonde 1A, 1B introduite dans le nez du patient 2, de maintenir la jupe 7 plaquée contre la paroi interne 4A de la narine 4 correspondante. En effet, les jets de gaz respiratoire, qui s'échappe des orifices latéraux 8, écartent radialement la jupe 7 en direction de la paroi interne 4A. En d'autres termes, le gaz respiratoire traversant les orifices 8 étire la jupe 7, qui se déforme alors légèrement pour venir s'appliquer avec justesse contre la paroi interne 4A correspondante. De cette façon, l'étanchéité entre la paroi interne 4A de la narine 4 et la jupe latérale 7 correspondante est assurée.

En outre, dans le premier exemple de réalisation illustré sur les figures 1 à 4, les orifices latéraux 8 favorisent la création de turbulences 9, de sorte que les jets gazeux G sortant des conduits 3 sont brassés et atteignent la muqueuse des parois internes 4A des narines 4 de façon diffuse, ce qui réduit sensiblement le risque de lésions de la muqueuse.

Dans ce premier exemple, chaque jupe latérale 7, ouverte à son extrémité distale 7B, se prolonge au-delà de l'extrémité distale 3A du conduit 3 correspondant, dans une direction longitudinale parallèle à l'axe longitudinal L-L dudit conduit 3. Autrement dit, la jupe 7 entoure intégralement l'extrémité distale 3A du conduit 3. De cette façon, la muqueuse de la narine 4 correspondante est isolée, de sorte qu'elle n'est pas traumatisée par les éventuels heurts ou frottements de l'extrémité 3A du conduit 3, la jupe latérale 7 formant une interface souple de protection.

En outre, comme le montrent les figures 1 à 4, l'extrémité proximale 7A de chaque jupe latérale 7 est rapportée (par exemple par collage) au conduit 3 correspondant par l'intermédiaire d'une collerette annulaire 10A (de faibles dimensions), qui participe à la formation de l'espace annulaire 11 autour du conduit 3, entre la jupe 7 et ce dernier. Cet espace annulaire 11 permet :
- d'éviter le collage de la jupe 7 sur la surface latérale extérieure de la partie du conduit 3 en regard de celle-ci ;
- de faciliter le passage du gaz (vicié et/ou respiratoire) au travers des orifices 8 ; et
- de contribuer à la protection de la muqueuse en maintenant l'extrémité distale 3A du conduit 3 éloignée de celle-ci.

Par ailleurs, dans le second exemple de réalisation représenté sur les figures 5 à 7, l'extrémité distale 7B de chaque jupe latérale 7 est rapportée (par exemple par collage) au conduit 3 correspondant par l'intermédiaire d'une collerette annulaire 10B (maintenue entre l'extrémité distale 3A du conduit 3 et les orifices 8), qui participe à la formation de l'espace annulaire 11 autour du conduit 3, entre la jupe 7 et ce dernier. Cet espace annulaire 11 procure les mêmes effets que ceux décrits ci-dessus (en relation avec la sonde 1A).

Dans ce second exemple, l'extrémité distale 7B de chacune des jupes 7 est fermée et est agencée au voisinage de l'extrémité distale 3A du conduit 3 correspondant.

Autrement dit, le gaz respiratoire et/ou le gaz vicié traversant les orifices 8 étirent la jupe 7 correspondante, en la déformant légèrement pour qu'elle s'applique contre la paroi interne 4A en vis-à-vis, pour ensuite être rejetés à l'extérieur de l'espace interne 11 (et donc de la narine 4 correspondante), au niveau de l'extrémité proximale ouverte 7A de la jupe 7.

En outre, en cas de surpression gazeuse à l'intérieur d'une narine 4, du gaz en surpression peut venir décoller la jupe latérale 7 correspondante appliquée contre la paroi interne 4A en regard, de manière à former un passage lui permettant de s'échapper vers l'extérieur, abaissant ainsi la pression à l'intérieur de la narine 4.

En d'autres termes, la sonde 1B permet de réguler automatiquement et de façon autonome la pression à l'intérieur des narines 4, ce qui prévient toute surpression susceptible d'entrainer un inconfort, voire même des lésions de la muqueuse, chez le patient.

Par ailleurs, la sonde nasale 1A comporte des moyens de prélèvement 12 de gaz vicié expiré par le patient 2, qui sont destinés à être reliés à des dispositifs de mesure (non représentés sur les figures) tels qu'un analyseur de CO2 (afin, par exemple, d'arrêter l'administration de gaz respiratoire dans le cas d'une détection de CO2 supérieure à un seuil prédéfini), un manomètre, etc.

Dans l'exemple de réalisation, les moyens de prélèvement 12 se présentent sous la forme de saillies tubulaires prolongeant le conduit de liaison 5, de part et d'autre de ses extrémités longitudinales, avec lequel elles communiquent.

En outre, comme le montre la figure 8 qui constitue un agrandissement de la zone G de la figure 6, un canalicule 13 est ménagé dans la paroi tubulaire d'un des conduits 3. Il débouche, à une de ses extrémités, au niveau de l'extrémité distale 3A du conduit 3 correspondant et est relié, à son autre extrémité, aux moyens de prélèvement 12. Ainsi, le prélèvement de gaz (et en particulier de CO2) est effectué directement dans la narine 4 correspondante.

Par ailleurs, pour améliorer le maintien de la sonde dans les narines d'un patient ainsi que l'étanchéité entre la paroi interne des narines et les jupes latérales, ces dernières peuvent avantageusement présenter une section transversale, par rapport à l'axe longitudinal L-L des conduits respectifs, au moins égale à la section transversale des narines. Ainsi, chaque jupe latérale souple, une fois insérée dans la narine correspondante du patient, est en contact avec la paroi interne de celle-ci.

On notera également que, pour réduire le bruit engendré par le passage des gaz dans les canaux 3B des conduits 3, il est envisageable d'introduire, dans chacun des canaux 3B, un ou plusieurs cylindres de mousse à cellules ouvertes perméable aux gaz, dont la section transversale est sensiblement égale à celle des canaux 3B.

## Revendications

1. Sonde nasale (1A, 1B) pour acheminer du gaz respiratoire dans l'appareil respiratoire d'un patient (2) comportant :
- deux conduits (3) destinés à être introduits, au moins partiellement, dans les narines (4) respectives du nez du patient (2) et aptes à être alimentés par du gaz respiratoire (G) ; et
- deux jupes latérales (7) souples dont chacune d'elles entoure un desdits conduits (3), sur au moins une partie de sa longueur, en ménageant un espace annulaire (11) entre elle et ledit conduit (3) correspondant, lesdites jupes latérales (7) étant destinées à être introduites, au moins en partie, dans les narines (4) correspondantes du nez du patient (2),
**caractérisée :**
- **en ce que** chacun desdits conduits (3) comporte au moins un orifice latéral (8) qui est ménagé dans sa paroi latérale et qui est en regard de ladite jupe latérale (7) associée ; et
- **en ce que** lesdites jupes latérales (7) sont déformables sous l'effet d'un jet de gaz s'échappant dudit au moins un orifice latéral (8), de manière à être appliquées contre la paroi interne des narines (4) correspondantes pour participer au maintien de ladite sonde nasale (1A, 1B) dans ces dernières.

2. Sonde selon la revendication 1,
**caractérisée en ce que** chacune desdites jupes latérales (7) entoure l'extrémité distale (3A) dudit conduit (3) associé.

3. Sonde selon l'une des revendications 1 ou 2,
**caractérisée en ce que** chacune desdites jupes latérales (7) est fabriquée en silicone.

4. Sonde selon l'une des revendications 1 à 3,
**caractérisée en ce que** chacun desdits conduits (3) comporte plusieurs orifices latéraux (8).

5. Sonde selon la revendication 4,
**caractérisée en ce que** lesdits orifices latéraux (8) de chacun desdits conduits (3) sont répartis équi-angulairement autour d'au moins une section dudit conduit (3).

6. Sonde selon l'une des revendications 1 à 5,
**caractérisée en ce que**, pour chacun desdits conduits (3), ledit espace annulaire (11) associé est ménagé au moyen d'une collerette annulaire (10A, 10B), appartenant à la jupe latérale (7) correspondante, qui est rapportée sur la surface latérale extérieure dudit conduit (3) associé.

7. Sonde selon la revendication 6,
**caractérisée en ce que** chaque collerette (10A) est liée à l'extrémité proximale (7A) de la jupe latérale (7) correspondante.

8. Sonde selon la revendication 6,
**caractérisée en ce que** chaque collerette (10B) est liée à l'extrémité distale (7B) de la jupe latérale (7) correspondante.

9. Sonde selon l'une des revendications 1 à 8,
**caractérisée en ce que** lesdits conduits (3) sont reliés l'un à l'autre par l'intermédiaire d'un conduit de liaison (5), lui-même relié à un embout de raccordement (6).

10. Sonde selon l'une des revendications 1 à 9,
**caractérisée en ce qu'**elle comporte des moyens (12, 13) de prélèvement de gaz vicié expiré par le patient (2), qui sont destinés à être reliés à des dispositifs de mesure.

## Patentansprüche

1. Nasale Sonde (1A, 1B) zum Zuführen von Atemgas in das Atemgerät eines Patienten (2), die
- zwei Leitungen (3), die dazu bestimmt sind, wenigstens teilweise in die jeweiligen Nasenlöcher (4) der Nase des Patienten (2) eingeführt zu werden, und die dazu ausgelegt sind, mit dem Atemgas (G) versorgt zu werden, und
- zwei flexible seitliche Abdeckungen (7), von denen jede eine der Leitungen (3) wenigstens auf einem Teil von deren Länge umgibt und dabei zwischen sich und der entsprechenden Leitung (3) einen ringförmigen Raum (11) einrichtet, wobei die seitlichen Abdeckungen (7) dazu bestimmt sind, wenigstens teilweise in die entsprechenden Nasenlöcher (4) der Nase des Patienten (2) eingeführt zu werden,
aufweist,
**dadurch gekennzeichnet,**
- **daß** jede der Leitungen (3) wenigstens ein seitliches Loch (8) aufweist, das in deren Wandung ausgeführt ist und das der zugehörigen seitlichen Abdeckung (7) gegenüber liegt, und
- **daß** die seitlichen Abdeckungen (7) unter dem Einfluß eines aus dem wenigstens einen seitlichen Loch (8) entweichenden Gasstrahls verformbar sind, so daß sie gegen die innere Wandung der entsprechenden Nasenlöcher (4) gedrückt werden, um am Festhalten der Nasensonde (1A, 1B) in letzteren mitzuwirken.

2. Sonde gemäß Anspruch 1, **dadurch gekennzeichnet, daß** jede der seitlichen Abdeckungen (7) das distale Ende (3A) der zugehörigen Leitung (3) umgibt.

3. Sonde gemäß einem der Ansprüche 1 oder 2, **dadurch gekennzeichnet, daß** jede der seitlichen Abdeckungen (7) aus Silikon hergestellt ist.

4. Sonde gemäß einem der Ansprüche 1 bis 8, **dadurch gekennzeichnet, daß** jede der Leitungen (3) mehrere seitliche Löcher (8) aufweist.

5. Sonde gemäß Anspruch 4, **dadurch gekennzeichnet, daß** die seitlichen Löcher (8) jeder der Leitungen (3) unter gleichen Winkeln um wenigstens einen Abschnitt der Leitung (3) herum angeordnet sind.

6. Sonde gemäß einem der Ansprüche 1 bis 5, **dadurch gekennzeichnet, daß** für jede der Leitungen (3) der zugehörige ringförmige Raum (11) mittels eines ringförmigen Kragens (10A, 10B) eingerichtet ist, der zur entsprechenden seitlichen Abdeckung (7) gehört und der auf der äußeren seitlichen Oberfläche der zugehörigen Leitung (3) angebracht ist.

7. Sonde gemäß Anspruch 6, **dadurch gekennzeichnet, daß** jeder Kragen (10A) mit dem proximalen Ende (7A) der zugehörigen seitlichen Abdeckung (7) verbunden ist.

8. Sonde gemäß Anspruch 6, **dadurch gekennzeichnet, daß** jeder Kragen (10B) mit dem distalen Ende (7B) der zugehörigen seitlichen Abdeckung (7) verbunden ist.

9. Sonde gemäß einem der Ansprüche 1 bis 8, **dadurch gekennzeichnet, daß** die Leitungen (3) durch eine Verbindungsleitung (5) miteinander verbunden sind, die ihrerseits mit einem Anschlußstück (6) verbunden ist.

10. Sonde gemäß einem der Ansprüche 1 bis 9, **dadurch gekennzeichnet, daß** sie Mittel (12, 13) zum Entnehmen von verbrauchtem, vom Patienten (2) ausgeatmetem Gas aufweist, die dazu bestimmt sind, mit Meßvorrichtungen verbunden zu sein.

## Claims

1. A nasal probe (1A, 1B) to convey respiratory gas into the respiratory system of a patient (2), including:
- two ducts (3) intended to be introduced, at least partially, into the respective nostrils (4) of the nose of the patient (2) and adapted to be supplied with respiratory gas (G); and
- two lateral flexible skirts (7), each of which is surrounding one of said ducts (3), over at least a portion of its length, by providing an annular space (11) between it and said corresponding duct (3), said lateral skirts (7) being intended to be introduced, at least in part, into the corresponding nostrils (4) of the nose of the patient (2),
**characterized:**
- **in that** each of said ducts (3) includes at least one lateral orifice (8) that is provided in its lateral wall and that is opposite said associated lateral skirt (7); and
- **in that** said lateral skirts (7) are deformable under the effect of a jet of gas escaping from said at least one lateral orifice (8), so as to be applied against the internal wall of the corresponding nostrils (4) to participate to the holding of said nasal probe (1A, 1B) within these latter.

2. The probe according to claim 1, **characterized in that** each of said lateral skirts (7) surrounds the distal end (3A) of said associated duct (3).

3. The probe according to one of claims 1 or 2, **characterized in that** each of said lateral skirts (7) is made of silicone.

4. The probe according to one of claims 1 to 3, **characterized in that** each of said ducts (3) includes several lateral orifices (8).

5. The probe according to claim 4, **characterized in that** said lateral orifices (8) of each of said ducts (3) are distributed equiangularly around at least one section of said duct (3).

6. The probe according to one of claims 1 to 5, **characterized in that**, for each of said ducts (3), said associated annular space (11) is provided by means of an annular flange (10A, 10B), belonging to the corresponding lateral skirt (7), that is added to the external lateral surface of said associated duct (3).

7. The probe according to claim 6, **characterized in that** each flange (10A) is connected to the proximal end (7A) of the corresponding lateral skirt (7).

8. The probe according to claim 6, **characterized in that** each flange (10B) is connected to the distal end (7B) of the corresponding lateral skirt (7).

9. The probe according to one of claims 1 to 8, **characterized in that** said ducts (3) are connected to each other through a connecting duct (5), itself connected to a connection top (6).

10. The probe according to one of claims 1 to 9, **characterized in that** it includes means (12, 13) for sampling foul gas exhausted by the patient (2), which are intended to be connected to measurement devices.
